# EUROPÄISCHE PATENTSCHRIFT

(11) **EP 0 251 111 B1**
(45) Veröffentlichungstag und Bekanntmachung des Hinweises auf die Patenterteilung: **27.01.1993**
(21) Anmeldenummer: 87108938.9
(22) Anmeldetag: 23.06.1987
(51) Int. Cl.: C07C 45/54, C07C 49/395, C07C 49/403, C07C 49/413

(54) **Verfahren zur Herstellung zyklischer Ketone**
Method for the production of cyclic ketones
Procédé pour la fabrication de cétones cycliques

(30) Priorität: 01.07.1986 DE 3622012
(43) Veröffentlichungstag der Anmeldung: 07.01.1988
(73) Patentinhaber: BASF Aktiengesellschaft, 67063 Ludwigshafen (DE)
(72) Erfinder: Decker, Martin, Dr., D-6700 Ludwigshafen (DE); Fischer, Rolf, Dr., D-6900 Heidelberg (DE); Franzischka, Wolfgang, Dr., D-6710 Frankenthal (DE); Kummer, Rudolf, Dr., D-6710 Frankenthal (DE); Schneider, Heinz-Walter, Dr., D-6700 Ludwigshafen (DE); Vagt, Uwe, Dr., D-6720 Speyer (DE)

(56) Entgegenhaltungen:
- DE-B- 1 768 138
- DE-C- 1 025 872
- US-A- 2 863 923
- CHEMICAL ABSTRACTS, Band 61, Nr. 11, 1964, Spalte 13179a-d, Columbus, Ohio, US; M.B. TUROVA-POLYAK et al.: "Ketones from acids in the presence of neodymium and erbium oxides"
- HOUBEN-WEYL, "Methoden der organischen Chemie", Band VII/2a, 1973, Ketone, Teil 1, S. 638, 634
- THE CHEMISTRY OF THE CARBONYL GROUP, S. Patai, Interscience Publishers, 1966, S. 217
- COMPT. REND., 158, 1914, S. 987-989

## Beschreibung

Diese Erfindung betrifft ein Verfahren zur Herstellung zyklischer Ketone durch Umsetzung aliphatischer Dicarbonsäureester an festen, oxidischen Katalysatoren in der Gas- oder Flüssigphase.

Aus der US-A-2 863 923 ist ein Verfahren zur Herstellung von 2,5-Dimethylcyclopentanon in Gegenwart von Metalloxiden bei Temperaturen von 350 bis 600°C bekannt. Bekanntlich läßt sich Cyclopentanon durch Erhitzen von Adipinsäure in Gegenwart von katalytischen Mengen Schwermetallsalzen in der Flüssigphase herstellen. Als Metalle werden hierbei z.B. Barium oder Thorium (für Thoriumoxid siehe DE-B-17 68 18) verwendet (siehe z.B.: Houben-Weyl, Methoden der organischen Chemie, Band VII/2a, Teil 1, Seiten 637-639 (1973). Diese Methode hat den Nachteil, daß bei den erforderlichen hohen Temperaturen Korrosionsprobleme auftreten und Schwermetalle benötigt werden.

Es war deshalb nach einem wirtschaftlich attraktiven und technisch einfach durchführbaren Verfahren zur Herstellung cyclischer Ketone, wie Cyclopentanon zu suchen.

Nach dem neuen Verfahren, das diese Anforderungen erfüllt, stellt man zyklische Ketone der Formel 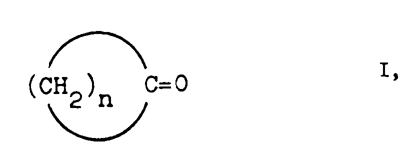
in der n eine ganze Zahl von 4 bis 6 bedeutet, dadurch her, daß man aliphatische Dicarbonsäureester der Formel

R¹OOC-(CH₂)ₙ-COOR² II,

in der n die obengenannte Bedeutung hat und R¹ und R² für Alkylreste mit 1 bis 12 Kohlenstoffatomen, Cycloalkylreste mit 5 oder 6 Kohlenstoffatomen, Aralkyl- oder Arylreste stehen und R² zusätzlich Wasserstoff bedeuten kann, bei Temperaturen von 300 bis 345°C an festen oxidischen Katalysatoren von Elementen der I. bis V. Hauptgruppe, der I. bis VIII. Nebengruppe des Periodensystems der Elemente oder Oxide der Seltenen Erdmetalle umsetzt.

Die erfindungsgemäße Reaktion läßt sich z.B. für die Umsetzung von Adipinsäuredimethylester zu Cyclopentanon durch die folgende Reaktionsgleichung darstellen:

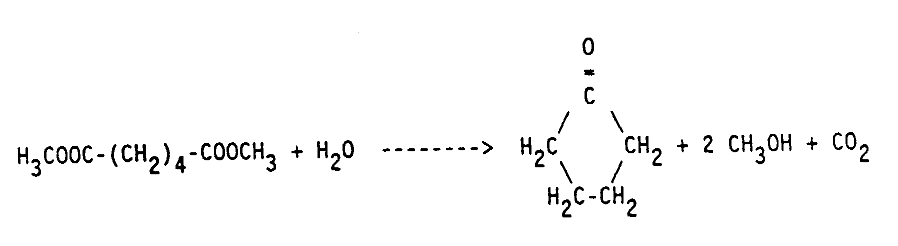

Es war bekannt, Adipinsäureester mit Hilfe stöchiometrischer Mengen starker Basen, wie Natriumalkoholaten oder Natriumamiden über das Reaktionszwischenprodukt Cyclopentanon-2-carbonsäureester in zwei Stufen in Cyclopentanon zu überführen (Dieckmann-Kondensation, s. Houben Weyl; Methoden der organischen Chemie, Band VIII, S. 574 (1952). Hierbei sind drei Verfahrensschritte (Kondensation, Neutralisation, Verseifung und Decarboxylierung) notwendig. Darüber hinaus muß die verwendete Base neutralisiert werden, so daß ein erheblicher Zwangsanfall an Neutralsalzen auftritt. Demgegenüber war es überraschend, daß man cyclische Ketone, wie Cyclopentanon nach dem erfindungsgemäßen Verfahren einstufig und dazu noch mit hohen Ausbeuten erhält.

Die als Ausgangsstoffe benötigten Ester der Formel II sind aliphatische, cycloaliphatische, araliphatische oder aromatische Mono- oder Diester der entsprechenden Dicarbonsäuren, wie Adipinsäure, Pimelinsäure oder Korksäure. Beispielhaft seien folgende Reste R¹ und R² genannt: Methyl-, Ethyl-, Propyl-, Isopropyl-, Butyl-, Isobutyl-, tert.-Butyl-, Hexyl-, Nonyl-, Dodecyl-, Cyclopentyl-, Cyclohexyl-, Phenyl- oder Benzylreste.

Beispielsweise können folgende Ester als Ausgangsstoffe verwendet werden: Adipinsäuredimethylester, Adipinsäuremonomethylester, Adipinsäurediethylester, Adipinsäuredibutylester, Adipinsäuredicyclohexylester, Adipinsäuredibenzylester, 1,7-Heptandisäuredimethylester, 1,7-Heptandisäurediethylester, 1,7-Heptandisäuremonomethylester, 1,7-Heptandisäuredibutylester, 1,7-Heptandisäuredicyclohexylester, 1,7-Heptandisäuredibenzylester, 1,8-Octandisäuredimethylester, 1,8-Octandisäurediethylester, 1,8-Octandisäuremonomethylester, 1,8-Octandisäuredibutylester, 1,8-Octandisäuredicyclohexylester und 1,8-Octandisäuredibenzylester.

Als Katalysatoren verwendet man feste oxidische Katalysatoren. Das sind z.B. Oxide von Elementen der I. bis V. Hauptgruppe, der I. bis VIII. Nebengruppe des periodischen Systems der Elemente oder Oxide der Seltenen Erdmetalle oder Gemische der genannten Oxide. So sind beispielsweise Erdalkalioxide, wie Magnesiumoxid, Calciumoxid, Bariumoxid, weiterhin Bortrioxid, Aluminiumoxid, Siliciumdioxid, z.B. in Form von Kieselgel, Kieselgur oder Quarz, ferner Zinndioxid, Bismutoxid, Kupferoxid, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide oder Gemische derartiger Oxide geeignet. Die Katalysatoren können noch durch Aufbringen von Zusätzen, wie Säuren (z.B. Phosphorsäure) oder Basen (z.B. Natriumhydroxid) modifiziert werden. Bevorzugt sind Magnesiumoxid, Bortrioxid, Aluminiumoxid, Siliciumdioxid, Zinkoxid, Titandioxid oder deren Gemische, von denen Aluminiumoxid-Katalysatoren ganz besonders geeignet sind.

Es ist zwar möglich, die erfindungsgemäße Umsetzung ohne Zusatz von Wasser durchzuführen, allerdings wird durch die Zugabe von Wasser eine bemerkenswerte Erhöhung von Selektivität und Standzeit erreicht. Das Molverhältnis von Ester II zu Wasser beträgt hierbei vorteilhaft 1 : 0,05 bis 1 : 20, insbesondere 1 : 0,1 bis 1 : 5.

Die Umsetzung kann in der Gasphase oder in der flüssigen Phase, gegebenenfalls auch unter Mitverwendung von Verdünnungsmitteln durchgeführt werden. Als Verdünnungsmittel sind z.B. Lösungsmittel geeignet, die unter den Reaktionsbdingungen vollständig oder weitgehend inert sind, z.B. Ether wie Dioxan oder Tetrahydrofuran. Vorzugsweise wird in der Gasphase gearbeitet, sofern gut verdampfbare Ausgangsstoffe vorliegen.

Die Umsetzung kann diskontinuierlich oder kontinuierlich als Festbettreaktion mit Festbettkatalysatoren, beispielsweise in Sumpf- oder Rieselfahrweise in der Flüssig- oder Gasphase oder als Wirbelbettreaktion mit in auf- und abwirbelnder Bewegung befindlichen Katalysatoren in der Gasphase oder aber mit in der Flüssigphase suspendierten Festbettkatalysatoren durchgeführt werden.

Die Umsetzung findet bei Temperaturen von 300 bis 345°C statt. Im allgemeinen wird die Reaktion unter Atmosphärendruck durchgeführt. Es ist Jedoch auch möglich, schwach verminderten oder schwach erhöhten Druck, z.B. bis zu 20 bar anzuwenden. Die Katalysatorbelastung liegt im allgemeinen bei 0,01 bis 40, vorzugsweise 0,1 bis 20 g Ester II je Gramm Katalysator und Stunde.

Die Umsetzung in der Flüssigphase wird beispielsweise so durchgeführt, daß man ein Gemisch aus dem Ester und gegebenenfalls Wasser in Gegenwart eines suspendierten Festbettkatalysators auf die gewünschte Reaktionstemperatur erhitzt. Nach Ablauf der notwendigen Reaktionszeit wird das Reaktionsgemisch abgekühlt und der Katalysator, z.B. durch Filtration, entfernt. Das Reaktionsgemisch wird anschließend zur Gewinnung des Ketons bzw. des unumgesetzten Esters fraktionierend destilliert.

Eine bevorzugte Ausführungsform des erfindungsgemäßen Verfahrens in der Gasphase sieht z.B. so aus, daß man ein Gemisch aus dem Ester und Wasser zunächst verdampft und dann, gegebenenfalls zusammen mit einem Inertgas, wie Stickstoff, Kohlendioxid oder Argon, bei der gewünschten Reaktionstemperatur gasförmig in eine in auf- und abwirbelnder Bewegung befindliche Katalysatorschicht einleitet. Der Reaktionsaustrag wird mittels geeigneter Kühlvorrichtungen kondensiert und anschließend durch fraktionierende Destillation aufgearbeitet. Unumgesetzter Ester kann zurückgeführt werden.

Das erfindungsgemäße Verfahren zur Herstellung der zyklischen Ketone, wie Cyclopentanon besitzt gegenüber den bekannten Verfahren den Vorteil, daß man das Produkt in einem Reaktionsschritt aus den einfach zugänglichen Estern mit hoher Ausbeute und Selektivität erhält.

Die nach dem erfindungsgemäßen Verfahren erhältlichen zyklischen Ketone sind wertvolle Zwischenprodukte. So läßt sich z.B. aus Cyclopentanon durch reduktive Aminierung Cyclopentylamin herstellen, das für die Synthese von Pflanzenschutzmitteln und Pharmazeutika von Interesse ist.

### Beispiel 1

Pro Stunde wurden 10 ml Adipinsäuredimethylester (DMA) in einen Verdampfer gepumpt und von dort gasförmig zusammen mit 3 l Stickstoff bei 300 bis 345°C über 15 ml des in der Tabelle I bezeichneten Katalysators geleitet. Die gasförmigen Reaktionsausträge wurden in Kühlfallen kondensiert, gewogen und gaschromatografisch analysiert. In der Tabelle I ist die Zusammensetzung der Reaktionsausträge in Abhängigkeit von der Temperatur nach jeweils 4 h Versuchszeit angegeben.

**Tabelle I**

| Nr. | Katalysator | Temperatur [°C] | Cyclopentanon [mol.-%] | DMA [mol.-%] |
|---|---|---|---|---|
| 1 | 10 g γ-Aluminiumoxid | 320 | 48 | 38 |

### Beispiel 2

Pro Stunde wurden 13 ml eines Gemisches aus DMA, Methanol und Wasser (78,5 Gew.-% DMA, 13,5 Gew.-% Methanol und 8 Gew.-% Wasser, Molverhältnis DMA : H₂O = 1 : 1) in einen Verdampfer gepumpt und von dort zusammen mit 3 l Stickstoff bei Temperaturen von 300 bis 345°C über 15 ml Katalysator geleitet. Die gasförmigen Reaktionsausträge wurden in Kühlfallen kondensiert, gewogen und gaschromatografisch analysiert. In der Tabelle II sind die verwendeten Katalysatoren sowie die Zusammensetzung der Reaktionsausträge angegeben.

**Tabelle 2**

| Nr. | Katalysator | Temperatur [°C] | Cyclopentanon [mol.-%] | DMA [mol.-%] |
|---|---|---|---|---|
| 1 | 10 g γ-Aluminiumoxid | 340 | 74 | 16 |

### Beispiel 3

Pro Stunde wurden 100 ml DMA bei 300°C verdampft und mit 30 l Stickstoff über 200 g (300 ml) γ-Aluminiumoxid (4-mm-Stränge), (Reaktionstemperatur 320°C) geleitet. Der gasförmige Reaktionsaustrag wurde über eine Versuchszeit von 7,5 h in Kühlfallen kondensiert (Gesamtzulauf-DMA: 805 g; Gesamtaustrag: 569 g; Zusammensetzung des Austrages nach quantitativer GC-Analyse: 32 Gew.-% Cyclopentanon, 52 Gew.-% DMA) und anschließend fraktionierend destilliert.

Auf diese Weise isolierte man 162 g (42 % d.Th.) reines Cyclopentanon vom Sdp. 129 bis 131°C. Zusätzlich wurden noch 297 g (37 % d.Th.) unumgesetztes DMA vom Sdp. 73 bis 85°C/2 mbar zurückerhalten.

### Beispiel 4

Pro Stunde wurden 10 ml Adipinsäuremonomethylester (Sdp. 162°C/10 mbar) in einen Verdampfer gepumpt und von dort gasförmig zusammen mit 3 l Stickstoff bei 320°C über 10 g (15 ml) γ-Aluminiumoxid (4-mm-Stränge) geleitet. Der gasförmige Reaktionsaustrag wurde über eine Versuchszeit von 10 h in Kühlfallen kondensiert, gewogen und gaschromatografisch analysiert. (Gesamtzulauf an Monoester: 109 g; Gesamtaustrag: 79 g; Zusammensetzung des Austrags: 42 Gew.-% Cyclopentanon (entsprechend einer Ausbeute von 58 % d.Th.), 30 Gew.-% DMA und 4 Gew.-% Monoester).

### Beispiel 5

Pro Stunde wurden 13 ml eines Gemisches aus DMA, Methanol und Wasser - (78,5 Gew.-% DMA, 13,5 Gew.-% Methanol und 8 Gew.-% Wasser, Molverhältnis DMA : H₂O = 1 : 1) in einen Verdampfer gepumpt und von dort zusammen mit 3 l Stickstoff in vier Versuchsreihen bei 300, 320, 330 und 340°C über 10 g (15 ml) γ-Aluminiumoxid (4-mm-Stänge) geleitet. Die gasförmigen Reaktionsausträge wurden in Kühlfallen kondensiert, gewogen und gaschromatografisch analysiert. Nach Versuchsende wurden die Austräge der einzelnen Versuchsreihen jeweils vereinigt und gaschromatografisch analysiert. Die Tabelle III zeigt eine Zusammenfassung der Ergebnisse.

**Tabelle III**

| Vers.-Nr. | Temp. [°C] | Versuchszeit [h] | DMA-Zulauf [g] | Austrag [g] | Cyclopentanon [mol-%] | DMA [mol-%] | Umsatz [%] | Sel. [%] |
|---|---|---|---|---|---|---|---|---|
| 1 | 300 | 168 | 1778 | 1963 | 24 | 66 | 34 | 71 |
| 2 | 320 | 216 | 2366 | 2533 | 30 | 59 | 41 | 73 |
| 3 | 330 | 192 | 1805 | 1571 | 60 | 29 | 71 | 85 |
| 4 | 340 | 72 | 642 | 464 | 49 | 11 | 89 | 55 |

Der Reaktionsaustrag von Vers.-Nr. 2 (320°C) wurde folgendermaßen aufgearbeitet: Zunächst wurden im Vakuum die leichtersiedenden Anteile (Cyclopentanon, Methanol, Wasser) abgezogen. Nach Zugabe von NaCl wurde die wäßrige Phase abgetrennt und die organische Phase bei Normaldruck fraktionierend destilliert. Ausbeute nach Destillation: 211 g (19 9 d.Th.) Cyclopentanon.

Der Reaktionsautrag von Vers.-Nr. 3 (330°C) wurde folgendermaßen aufgearbeitet: Zunächst wurden im Vakuum die leichtersiedenden Anteile (Cyclopentanon, Methanol, Wasser) abgezogen und mit Dichlormethan versetzt. Die organische Phase wurde abgetrennt und die wäßrige Phase noch dreimal mit wenig Dichlormethan gewaschen. Die vereinigten organischen Phasen wurden durch Auskreisen des Wassers mit Dichlormethan weitgehend getrocknet und anschließend bei Normaldruck fraktionierend destilliert. Ausbeute nach Destillation: 470 g (54 % d.Th.) Cyclopentanon.

### Beispiel 6

Pro Stunde wurden 150 ml DMA und 33 ml Wasser (Molverhältnis DMA : H₂O = 1 : 2) bei ca. 300°C verdampft und mit 30 l Stickstoff über 200 g (300 ml) γ₋Aluminiumoxid (4 mm Stränge; Katalysator aus Versuch 6) (Reaktionstemperatur 320°C) geleitet. Der gasförmige Reaktionsaustrag wurde über eine Versuchszeit von 8 h in Kühlfallen kondensiert. (Gesamtzulauf-DMA: 1208 g; Gesamtaustrag: 1069 g; Zusammensetzung des Austrages nach quantitativer GC-Analyse: 31 Gew.-% Cyclopentanon, 26.5 Gew.-% DMA).

Zur Aufarbeitung wurden die leichtersiedenden Anteile des Reaktionsaustrages (Cyclopentanon, Wasser, Leichtsieder wie Dimethylether und Methanol) im Vakuum abgezogen und mit Dichlormethan versetzt. Die organische Phase wurde abgetrennt und die wäßrige Phase noch dreimal mit wenig Dichlormethan gewaschen. Die vereinigten organischen Phasen wurden durch Auskreisen des Wassers mit Dichlormethan weitgehend getrocknet und anschließend bei Normaldruck fraktionierend destilliert. Ausbeute nach Destillation: 300 g (51 % d.Th.) Cyclopentanon.

### Beispiel 7

Pro Stunde wurden 150 ml DMA und 33 ml Wasser (Molverhältnis DMA : H₂O = 1 : 2) bei ca. 300°C verdampft und mit 60 l Stickstoff über 300 g (300 ml) γ-Aluminiumoxid-Wirbelkontakt (Korngröße 0,14 - 0,315 mm) (Reaktionstemperatur 330°C geleitet. Der gasförmige Reaktionsaustrag wurde jeweils in Kühlfallen kondensiert und gaschromatografisch analysiert. Die Tabelle IV faßt die Ergebnisse zusammen.

**Tabelle IV**

| Vers.-Nr. | Temp. [°C] | Versuchszeit [h] | DMA-Zulauf [g] | Austrag [g] | Cyclopentanon [mol-%] | DMA [mol-%] | Umsatz [%] | Sel. [%] |
|---|---|---|---|---|---|---|---|---|
| 1 | 330 | 4,5 | 730 | 758 | 54 | 40 | 60 | 90 |

### Beispiel 8

Pro Stunde wurden 10 ml eines Gemisches aus 1,7-Hexandisäuredimethylester, Methanol und Wasser (79 Gew.-% Diester, 13,4 Gew.-% Methanol und 7,6 Gew.-% Wasser, Molverhältnis Diester: H₂O = 1 : 1) in einen Verdampfer gepumpt und von dort zusammen mit 3 Litern Stickstoff über 10 g (15 ml) Katalysator (Korngröße 0,5 mm) geleitet. Die gasförmigen Reaktionsausträge wurden in Kühlfallen kondensiert, gewogen und gaschromatografisch analysiert. Die Tabelle V zeigt die Ergebnisse.

**Tabelle V**

| Nr. | Temp. [°C] | Versuchszeit [h] | Diester-Zulauf [g] | Austrag [g] | Cyclohexanon [mol-%] | Diester [mol-%] | Umsatz [%] | Sel. [%] | Kat |
|---|---|---|---|---|---|---|---|---|---|
| 1 | 340 | 30 | 253 | 270 | 35 | 58 | 42 | 83 | A |
| Katalysator A: γ-Aluminiumoxid | | | | | | | | | |

### Beispiel 9

Pro Stunde wurden 10 ml eines Gemisches aus 1,8-Octandisäuredimethylester, Methanol und Wasser (71 Gew.-% Diester, 22,6 Gew.-% Methanol und 6,4 Gew.-% Wasser, Molverhältnis Diester : H₂O = 1 : 1) in einen Verdampfer gepumpt und von dort zusammen mit 3 Litern Stickstoff über 10 g (15 ml) Katalysator (Korngröße 0,5 mm) geleitet. Die gasförmigen Reaktionsausträge wurden in Kühlfallen kondensiert, gewogen und gaschromatografisch analysiert. In Tabelle VI sind die Ergebnisse zusammengefaßt.

**Tabelle VI**

| Nr. | Temp. [°C] | Versuchszeit [h] | Diester Zulauf [g] | Austrag [g] | Cycloheptanon [mol-%] | Diester [mol-%] | Umsatz [%] | Sel. [%] | Kat |
|---|---|---|---|---|---|---|---|---|---|
| 1 | 340 | 24 | 161 | 208 | 14 | 66 | 34 | 41 | A |
| Katalysator A: γ-Aluminiumoxid | | | | | | | | | |

### Beispiel 10

Pro Stunde wurden 50 ml 1,7-Hexandisäuredimethylester und 33 ml Wasser bei ca. 300°C verdampft und mit 100 l Stickstoff über 300 g (380 ml) γ₋Aluminiumoxid₋Wirbelkontakt, Korngröße 0,14 - 0,315 mm) (Reaktionstemperatur 345°C) geleitet. Der gasförmige Reaktionsaustrag wurde in Kühlfallen kondensiert und gaschromatografisch analysiert. Tabelle VII zeigt das Ergebnis.

**Tabelle VII**

| Versuchszeit [h] | Diester-Zulauf [g] | Austrag [g] | Cyclohexanon [mol-%] | Diester [mol-%] | Umsatz [%] | Sel. [%] |
|---|---|---|---|---|---|---|
| 6 | 287 | 453 | 85 | 12 | 88 | 96 |

### Beispiel 11

Pro Stunde wurden 50 ml 1,8-Octandisäuredimethylester und 33 ml Wasser bei ca. 300°C verdampft und mit 100 l Stickstoff über 300 g (380 ml) γ-Aluminiumoxid-Wirbelkontakt Korngröße 0,14 - 0,315 mm) (Reaktionstemperatur 345°C) geleitet. Der gasförmige Reaktionsaustrag wurde in Kühlfallen kondensiert und gaschromatographisch analysiert. Tabelle VII zeigt das Ergebnis.

**Tabelle VIII**

| Versuchszeit [h] | Diester-Zulauf [g] | Austrag [g] | Cycloheptanon [mol-%] | Diester [mol-%] | Umsatz [%] | Sel. [%] |
|---|---|---|---|---|---|---|
| 6 | 299 | 487 | 29 | 42 | 58 | 50 |

### Beispiel 12

In einem Wirbelschichtreaktor, der im unteren Teil eine Verteilerplatte für das Reaktionsgas und im oberen Teil eine Abscheidevorrichtung für Katalysatorstaub enthielt, wurden 750 Volumenteile (490 Gewichtsteile) γ-Aluminiumoxid der Körnung 0,06 bis 0,2 mm als Katalysator eingefüllt. Der Katalysator wurde mit 75 000 Volumenteilen Stickstoff aufgewirbelt und auf 340°C erhitzt. Je Stunde wurden 316 Gewichtsteile DMA und 66 Gewichtsteile Wasser im Stickstoffstrom bei 290°C verdampft und bei 340°C durch die Katalysatorschicht geleitet. Insgesamt wurden 3 240 Gewichtsteile DMA eingesetzt. Durch Kondensation und durch Auswaschen des Abgases mit Methanol wurden 3 179 Gewichtsteile Rohprodukt erhalten, in welchem 1 312 Cewichtsteile Cyclopentanon und 51 Gewichtsteile DMA neben Wasser und Methanol enthalten waren. Die Ausbeute, bezogen auf umgesetztes DMA, betrug 85,2 Mol-%.

## Patentansprüche

1. Verfahren zur Herstellung zyklischer Ketone der Formel 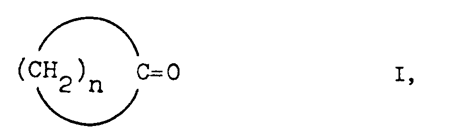 in der n eine ganze Zahl von 4 bis 6 bedeutet, dadurch gekennzeichnet, daß man aliphatische Dicarbonsäureester der Formel
R¹OOC-(CH₂)ₙ-COOR² II,
in der n die obengenannte Bedeutung hat und R¹ und R² für Alkylreste mit 1 bis 12 Kohlenstoffatomen, Cycloalkylreste mit 5 oder 6 Kohlenstoffatomen, Aralkyl- oder Arylreste stehen und R² zusätzlich Wasserstoff bedeuten kann, bei Temperaturen von 300 bis 345 ^{o}C an festen oxidischen Katalysatoren von Elementen der I. bis V. Hauptgruppe, der I. bis VIII. Nebengruppe des Periodischen Systems der Elemente oder Oxide der Seltenen Erdmetall umsetzt.

2. Verfahren nach Anspruch 1, dadurch gekennzeichnet, daß man als Katalysatoren Magnesiumoxid, Aluminiumoxid, Siliciumdioxid, Zinkoxid oder Titandioxid verwendet.

3. Verfahren nach Anspruch 1 und 2, dadurch gekennzeichnet, daß man als Katalysatoren Magnesiumoxid, Aluminiumoxid oder Siliciumdioxid verwendet.

4. Verfahren nach Anspruch 1 bis 3, dadurch gekennzeichnet, daß man als Katalysator Aluminiumoxid verwendet.

5. Verfahren nach den Ansprüchen 1 bis 4, dadurch gekennzeichnet, daß man die Umsetzung der Ester (II) unter Zusatz von Wasser durchführt, wobei ein Molverhältnis von II zu Wasser von 1 : 0,05 bis 1 : 20, insbesondere 1 : 0,1 bis 1 : 5 gewählt wird.

6. Verfahren nach den Ansprüchen 1 bis 5, dadurch gekennzeichnet, daß man die erfindungsgemäße Umsetzung im Wirbelbett durchführt.

## Claims

1. A process for the preparation of a cyclic ketone of the formula 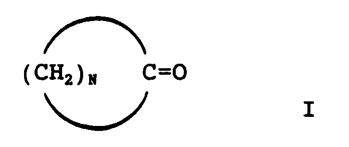 where n is an integer from 4 to 6, wherein an aliphatic dicarboxylate of the formula
R¹OOC-(CH₂)_{N}-COOR³ II
where n has the above meaning and R¹ and R² are each alkyl of 1 to 12 carbon atoms, cycloalkyl of 5 or 6 carbon atoms, aralkyl or aryl and R² may also be hydrogen, is converted at from 300 to 345°C over a solid oxide catalyst of an element of main groups I to V or of subgroups I to VIII of the periodic table of elements or an oxide of the rare earth metals.

2. A process as claimed in claim 1, wherein the catalyst used is magnesium oxide, alumina, silica, zinc oxide or titanium dioxide.

3. A process as claimed in claim 1 or 2, wherein the catalyst used is magnesium oxide, alumina or silica.

4. A process as claimed in any of claims 1 to 3, wherein the catalyst used is alumina.

5. A process as claimed in any of claims 1 to 4, wherein the conversion of the ester (II) is carried out with the addition of water, a molar ratio II to water of from 1:0.05 to 1:20, in particular from 1:0.1 to 1:5, being chosen.

6. A process as claimed in any of claims 1 to 5, wherein the reaction according to the invention is carried out in a fluidized bed.

## Revendications

1. Procédé de préparation de cétones cycliques de la formule 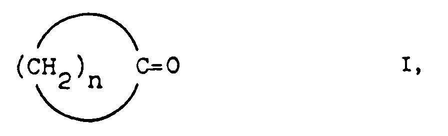 dans laquelle n représente un nombre entier de 4 à 6, caractérisé en ce que l'on fait réagir des esters d'acides dicarboxyliques aliphatiques de la formule
R¹OOC-(CH₂)ₙ-COOR² II,
dans laquelle n possède les significations qui lui ont été attribuées ci-dessus et R¹ et R² représentent des radicaux alkyle comportant de 1 à 12 atomes de carbone, des radicaux cycloalkyle comportant 5 ou 6 atomes de carbone, des radicaux aralkyle ou aryle et R² peut représenter de surcroît un atome d'hydrogène, à des températures de 300 à 345°C, sur des catalyseurs du type oxyde d'élements des premier à cinquième groupes principaux, des premier à huitième groupes secondaires du système périodique des éléments, ou des oxydes des métaux des terres rares.

2. Procédé suivant la revendication 1, caractérisé en ce qu'à titre de catalyseurs, on utilise de l'oxyde de magnésium, de l'oxyde d'aluminium, du dioxyde de silicium, de l'oxyde de zinc, ou du dioxyde de titane.

3. Procédé suivant les revendications 1 et 2, caractérisé en ce qu'on utilise de l'oxyde de magnésium, de l'oxyde d'aluminium, ou du dioxyde de silicium, à titre de catalyseurs.

4. Procédé suivant les revendications 1 à 3, caractérisé en ce que l'on utilise de l'oxyde d'aluminium à titre de catalyseur.

5. Procédé suivant les revendications 1 à 4, caractérisé en ce que l'on entreprend la réaction des esters (II) sous addition d'eau, où l'on choisit un rapport molaire de II à l'eau de 1:0,5 à 1:20, plus particulièrement de 1:0,1 à 1:5.

6. Procédé suivant les revendications 1 à 5, caractérisé en ce que l'on entreprend la réaction selon l'invention en lit turbulent ou fluidisé.
